Europäisches Patentamt

(19) European Patent Office

Office européen des brevets

(11) Publication number: **0 042 551**
**B1**

(12) # EUROPEAN PATENT SPECIFICATION

(45) Date of publication of patent specification: **28.11.84**

(51) Int. Cl.³: **A 61 N 1/04**

(21) Application number: **81104508.7**

(22) Date of filing: **11.06.81**

(54) **Electrode for cardiac stimulators.**

(30) Priority: **19.06.80 IT 6796380**

(43) Date of publication of application:
**30.12.81 Bulletin 81/52**

(45) Publication of the grant of the patent:
**28.11.84 Bulletin 84/48**

(84) Designated Contracting States:
**DE FR GB NL**

(56) References cited:
**DE-A-1 939 806**
**FR-A-2 235 708**
**US-A-3 749 101**
**US-A-4 011 861**
**US-A-4 026 303**
**US-A-4 156 429**

(73) Proprietor: **SORIN BIOMEDICA S.p.A.**
**Strada per Crescentino 31**
**I-13040 Saluggia (Vercelli) (IT)**

(72) Inventor: **Freud, Gerrit Ernst**
**Keizersgracht 694**
**NL-1017 EV Amsterdam (NL)**

(74) Representative: **Jacobacci, Filippo et al**
**c/o JACOBACCI-CASETTA & PERANI S.n.c. Via**
**Alfieri, 17**
**I-10121 Torino (IT)**

(56) References cited:
**BIOMED. TECHNIK, vol. 24, no. 1/2,**
**January/February 1979 Berlin, DE BISPING**
**"Übersicht über verankerbare transvenöse**
**Schrittmachersonden" pages 16-27**

The file contains technical information
submitted after the application was filed and
not included in this specification

Courier Press, Leamington Spa, England.

## Description

This invention refers to an electrode with a helical tip for cardiac stimulators ("pacemaker"), particularly suited for atrial applications.

Electrodes are known with a helical tip, e.g. from US—A—4026303, formed by a loop of metallic wire loosely twisted on itself so as to form helical congruent turns merging into each other at the free end of the tip. Such electrodes have the advantage of a "screwed" anchorage within the trabeculae of the cardiac wall with the consequent stability of the electrode. A further advantage seems to consist in the lowering of the threshold of stimulation.

The object of this invention is to provide an electrode with improved helical tip, which would be practically not-thrombogenic (particularly at the free end), would produce only an insignificant trauma in the endocardiac surface and in any case would not damage the myocardium. A further object is to obtain a particularly low stimulation threshold and a high sensitivity to the impulses coming from the cardiac wall.

According to the invention, the helical tip of the electrode is formed by a metal strip helically twisted about its longitudinal axis the peripheral edges and the free terminal end of the strip being rounded.

Preferably, the tip has a total of from 1 to 3 torsion turns. Advantageously, the length of the tip is of the order of 5—7 mm, the width of the strip is of the order of 1.0 to 1.5 and its thickness is of the order of 0.2 to 0.3 mm.

According to a preferred embodiment, the strip is constituted by a platinum/iridium alloy (preferably with Pt/Ir=90/10) and is coated with a thin layer of platinum sponge, so that the whole useful surface of the helical tip is porous preferably with pores of the diameter of about 40 microns. Such coating can easily be obtained by electrolytic deposition forming a porous deposit, and by a subsequent thermal treatment of sintering according to a process forming the subject matter of Applicant's separate application EP—A—0043461 filed on the same date and having the title "Electrode for cardiac stimulators and process for its manufacture".

An electrode according to the invention is illustrated by way of example in the appended drawings, in which:

Fig. 1 is an axial cross-sectional view of the terminal part of an endocardiac probe comprising the involved electrode and

Fig. 2 is a transverse cross-sectional view on line II—II of Fig. 1.

In the illustrated embodiment, the electrode comprises a body 1 in the form of a cylindrical cap and a helical tip 2, both of platinum/iridium 90/10. The cap-shaped body 1 is inserted into the end of a silicone rubber sheath 3 containing an electrical conductor 4 in form of helical spiral, which can be monofilament but preferably is composed of three filaments for reliability reasons, according to the known art. The spiral 4 penetrates into the cap-shaped body 1, which is crimped on a length of the spiral containing a small metallic cylinder 5 acting as "anvil" or "mandrel" against crushing of the spiral during crimping.

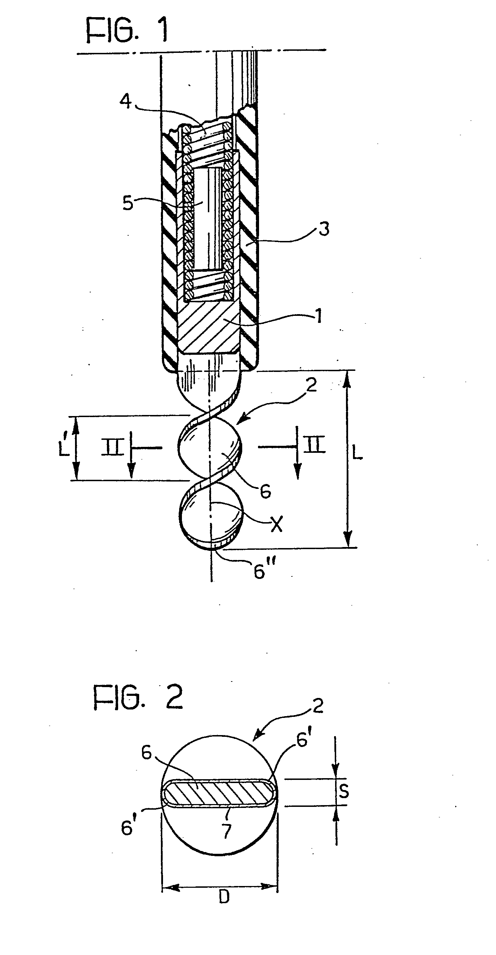

The helical tip 2 is formed by a strip 6 of Pt/Ir 90/10, which can be integral with the body 1 or fitted to the latter by welding. The peripheral edges (6') and the terminal end of the strip 6 are rounded, as is shown in Fig. 2. The width D of the strip 6 is equal to the diameter of the body 1 and amounts to about 1.2 mm. Its thickness S is about 0.25 mm. The free end 6" of the strip is rounded and the strip is twisted on its longitudinal axis X for a total of about 1.2 turns. The length L of the resulting helical tip is of 6—7 mm. The tip is entirely coated with a porous layer 7 of platinum, of a thickness of a few hundredths of millimeter.

According to a preferred embodiment of the invention, while the whole helical tip serves for anchoring in the cardiac wall, only a limited segment of its length is freely exposed and coated with a porous layer, while the remaining part of the tip is coated by an electrically insulating pellicular layer, so as to obtain on said segment the desired current density. Such segment is preferably an intermediate segment, as for example the half turn denoted by L' in Fig. 1. The pellicular insulating layer may have a thickness of only a few microns and can be obtained by means of a silicone rubber paint. However, the most suited material is that known under the trade name PARYLENE, which designates polymers based on paraxylylene forming thermoplastic films of polymerisation from vapour phase on the surface of to be coated, kept cold. The most suited type of Parylene seems to be the polymonochloro-p-xylylene, but also the ordinary poly-p-xylylene can be employed with success. During the deposition from vapour phase, the segment of the helical tip (as that L' in Fig. 1) which has to remain uncoated is kept protected by means of a suitable temporary "resist" layer, constituted for example by an ordinary self-adhesive tape.

## Claims

1. Electrode with helical tip (2) for cardiac stimulators, characterized in that the tip (2) is formed by a metallic strip (6) helically twisted about its longitudinal axis, the peripheral edges (6') and the free terminal end (6") of the strip (6) being rounded.

2. Electrode according to claim 1, in which the tip (2) has a total of from 1 to 3 complete turns of torsion.

3. Electrode according to claim 1 or 2, in which the length of the tip (2) is 5—7 mm.

4. Electrode according to claim 1, 2 or 3, in which the width of the strip (6) is 1.0—1.5 mm and its thickness is 0.2—0.3 mm.

5. Electrode according to any of the preceding claims, in which only a limited segment of the length of the helical tip (2) is freely exposed, the remaining part of the tip being coated with an electrically insulating pellicular layer.

6. Electrode according to claim 5, in which said limited segment is an intermediate one.

7. Electrode according to any of the preceding claims, in which at least the exposed segment of the helical tip has a porous surface.

8. Electrode according to claim 7, in which the helical tip is of platinum/iridium and the porous surface is constituted by platinum sponge.

9. Electrode according to claim 8, in which the weight ratio Pt/Ir amounts at least substantially to 90/10.

10. Electrode according to claim 8, in which the diameter of the pores of the platinum sponge is about 40 microns.

11. Electrode according to claim 8 or 10, in which the platinum sponge is a sintered electrolytic porous layer.

## Revendications

1. Electrode à pointe hélicoidale (2) pour stimulateurs cardiaques, caractérisé en ce que la pointe (2) est formée par une bande métallique (6) torsadée en hélice suivant son axe longitudinal et où les bords périphériques (6') et l'extrémité terminale libre (6'') de la bande (6) sont arrondis.

2. Electrode selon la revendication 1, dans laquelle la pointe (2) présente de 1 à 3 spires complètes de torsion au total.

3. Electrode selon la revendication 1 ou 2, dans laquelle la longueur de la pointe (2) est de 5 à 7 mm.

4. Electrode selon la revendication 1, 2 ou 3, dans laquelle la largeur de la bande (6) est de 1,0 à 1,5 mm et son épaisseur est de 0,2 à 0,3 mm.

5. Electrode selon l'une quelconque des revendications précédentes, dans laquelle seul un segment limité de la longueur de la pointe hélicoïdale (2) est librement exposé la partie restante de la pointe étant revêtue d'une pelliculaire électriquement isolante.

6. Electrode selon la revendication 5, dans laquelle ledit segment limité est un segment intermédiaire.

7. Electrode selon l'une quelconque des revendications précédentes, dans laquelle au moins le segment exposé de la pointe hélicoïdale possède une surface poreuse.

8. Electrode selon la revendication 7, dans laquelle la pointe hélicoïdale est faite de platine/irridium et la surface poreuse est constituée de mousse de platine.

9. Electrode selon la revendication 8, dans laquelle le rapport pondéral Pt/Ir est au moins sensiblement égal à 90/10.

10. Electrode selon la revendication 8, dans laquelle le diamètre des pores de la mousse de platine est d'environ 40 $\mu$m.

11. Electrode selon la revendication 8 ou 10, dans laquelle la mousse de platine est une couche poreuse électrolytique frittée.

## Patentansprüche

1. Herzschrittmacher-Elektrode mist schraubenförmiger Spitze, dadurch gekennzeichnet, daß die Spitze (2) von einem Metallstreifen (6) gebildet ist, der schraubenförmig um seine Längsachse gewunden ist, und daß die Seitenkanten (6') dieses Metallstreifens (6) und die Kante (6'') seines freien Endes abgerundet sind.

2. Elektrode nach Anspruch 1, dadurch gekennzeichnet, daß die Spitze (2) ingesamt 1 bis 3 vollständige Windungen besitzt.

3. Electrode nach Anspruch 1 dadurch gekennzeichnet, daß die Länge der Spitze (2) 5 bis 7 mm beträgt.

4. Elektrode nach Anspruch 1, 2 oder 3, dadurch gekennzeichnet, daß die Breite des Metallstreifens (6) 1,0 bis 1,5 mm und seine Dicke 0,2 bis 0,3 mm beträgt.

5. Elektrode nach einem der vorhergehenden Ansprüche dadurch gekennzeichnet, daß nur ein begrenzter Abschnitt der schraubenförmigen Spitze (6) freiliegt und daß der übrige Teil der Spitze mit einer filmartigen elektrisch isolierenden Schicht überzogen ist.

6. Elektrode nach Anspruch 5, dadurch gekennzeichnet, daß der begrenzte Abschnitt ein zwischenliegender Abschnitt ist.

7. Elektrode nach einem der vorhergehenden Ansprüche, dadurch gekennzeichnet, daß zumindest der freiliegende Abschnitt der schraubenförmigen Spitze eine poröse Oberfläche besitzt.

8. Elektrode nach Anspruch 7, dadurch gekennzeichnet, daß die schraubenförmige Spitze aus Platin/Iridium ist und die Poröse Oberfläche aus Platinmohr besteht.

9. Elektrode nach Anspruch 8, dadurch gekennzeichnet, daß das Gewichtsverhältnis Pt/Ir im wesentlichen wenigstens 90/10 beträgt.

10. Elektrode nach Anspruch 8, dadurch gekennzeichnet daß der Durchmesser der Poren des Platinmohrs ungefähr 40 Mikron beträgt.

11. Elektrode nach Anspruch 8 oder 10, dadurch gekennzeichnet, daß der Platinmohr aus einer porösen elektrolytischen gesinterten Schicht besteht.

FIG. 1

FIG. 2